# EUROPEAN PATENT APPLICATION

(11) **EP 0 950 425 A2**
(43) Date of publication of application: **20.10.1999**
(21) Application number: 99302205.2
(22) Date of filing: 22.03.1999
(51) Int. Cl.: A61M 25/00

(54) **Medico-surgical tubes and methods of manufacture**

(30) Priority: 15.04.1998 GB 9807856
(71) Applicant: Smiths Industries Public Limited Company, London, NW11 8DS (GB)
(72) Inventor: Batchelor, Kester Julian, Burnham-on-Sea, Somerset TA8 2RY (GB)
(74) Representative: Flint, Jonathan McNeill

(57) **Abstract**

An epidural catheter 1 with a soft tip 2 is made by co-extruding an inner layer 11 and an outer layer 12, the inner layer being of a stiffer plastics that the outer layer. The extrusion machine 20 has two extrusion heads 21 and 22, the one 21 for the inner layer 11 having a movable cone 29 that is moved in periodically to increase the internal diameter of inner layer and then to cut off flow of extrudate entirely. This produces a region 13 having a part 14 with a thinner inner layer 11 and part 15 where the inner layer is absent. The tubing 40 is cut at the end of these regions 13 which are then closed and formed with a side opening 3 so that the patient end tip 2 is less stiff than the remainder of the catheter.

## Description

This invention relates to methods of making medico-surgical tubes.

The invention is more particularly concerned with tubes with a soft tip, and with methods of manufacture of such tubes.

It is often desirable for medico-surgical tubes or catheters to have a soft tip, so as to reduce trauma caused by contact of the tip with patient tissue. In epidural catheters a soft tip reduces the risk that the catheter will damage the dura. Various arrangements have been proposed for providing a soft tip, such as by attaching or moulding onto the shaft of the catheter a separate component of a softer material. Such an arrangement is not entirely satisfactory because a separate assembly operation is needed to form the tip, leading to increased manufacturing expense. Also, there is always some risk that a separate component might become detached from the body of the catheter. Other arrangements in which the rear part of the catheter is reinforced can also be difficult to make by automated assembly, thereby making the catheter relatively expensive. In W094/01160 there is described an epidural catheter where an inner tube is preformed and then an outer tube is provided around it extending beyond the inner tube at one end.

It is an object of the present invention to provide an alternative medico-surgical tube and method of manufacture of a such a tube.

According to the present invention there is provided a method of making a medico-surgical tube, characterised in that a tubular member is formed by continuously co-extruding an inner layer and an outer layer, that the thickness of the extruded inner layer is periodically reduced to form regions along the tubing of reduced thickness, and that the tubing is cut at the regions to form tubes with an end region of reduced stiffness.

The thickness of the inner layer is preferably reduced by increasing its internal diameter. The thickness of the inner layer may be reduced along the region such that the stiffness varies along the region. The thickness of the inner layer is preferably reduced to zero along a part at least of the region. The material of the inner layer is preferably stiffer than the material of the outer layer. The cut end of the tubing including the region of reduced thickness may be end formed and a side opening formed close to the closed end.

An epidural catheter, a machine and a method of making an epidural catheter according to the present invention, will now be described, by way of example, with reference to the accompanying drawings, in which:
- Figure 1: is a side elevation view of the catheter;
- Figure 2: is an enlarged cross-sectional side elevation view of the patient end of the catheter;
- Figure 3: is a schematic diagram of a machine for making the catheter; and
- Figure 4: illustrates tubing produced by the machine of Figure 3.

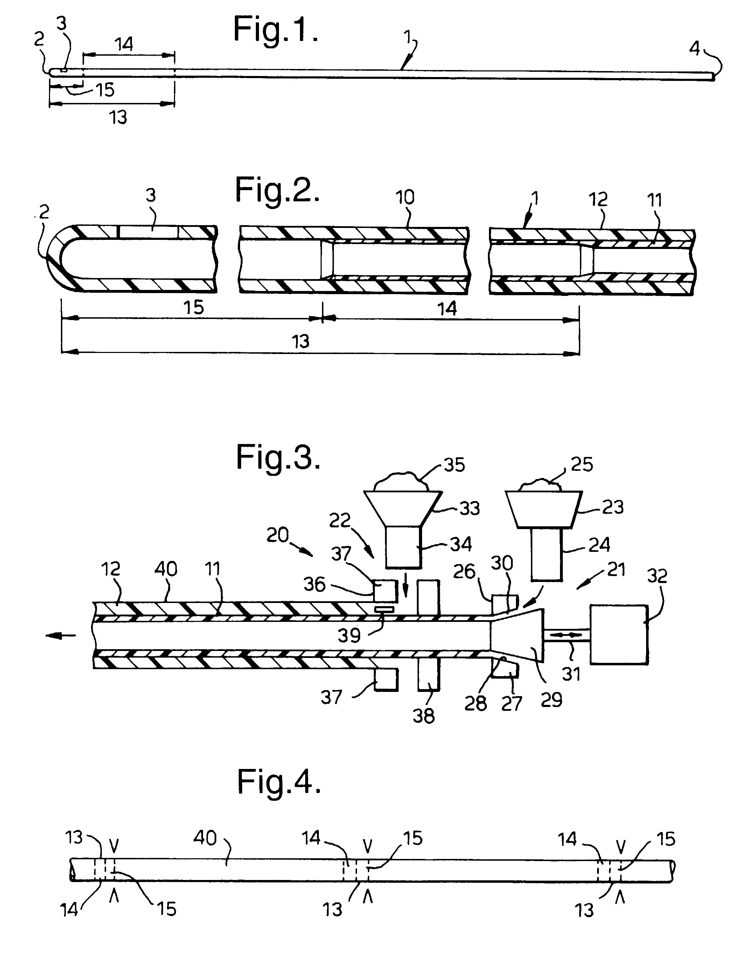

With reference first to Figure 1, the catheter 1 is about 75-100cm long with a rounded tip at its patient end 2 and a side opening 3 close to the tip. The machine end 4 of the catheter 1 is open and cut square for attachment to a conventional epidural connector, not shown.

Referring now also to Figure 2, the wall 10 of the catheter 1 is extruded from a thermoplastics material. The wall 10 has an inner layer 11 and an outer layer 12 of different materials, the inner layer 11 being of a stiffer material than the outer layer 12. Both layers 11 and 12 may be of the same polymer, such as PVC, but with differing amounts of plasticizer, or the layers could be of different polymers, such as an outer layer of PVC and an inner layer of ABS, nylon or polycarbonate. Towards the patient end 2 of the catheter 1, there is a region 13 about 10-15 cm long where the thickness of the inner layer 11 is reduced progressively. More particularly, the thickness of the inner layer 11 in the rear part 14 of the region 13 is about half that along the remaining, rear part of the catheter. In the forward part 15 of the region 13 the thickness of the inner layer 11 is reduced to zero so that, for about 2cm at the patient end tip 2, the catheter 1 does not have any inner layer.

The reduced thickness of the inner layer 11 along the region 13 makes this more flexible than the remainder of the catheter 1, and the tip 2, with no inner layer, is relatively soft. This reduces the risk of damage to the dura and enables the forward end of the catheter 1 to bend to conform to the shape of the epidural space with a reduced risk of kinking. Because the inner layer 11 is stiffer than the outer layer 12, it can be relatively thin and still provide sufficient rigidity to the main part of the catheter. This ensures that the inner diameter of the catheter 1 is kept as large as possible. Reducing the thickness of the inner layer 11, even to a zero thickness, does not greatly affect the overall thickness of the wall of the catheter 1 so that it still presents a relatively smooth internal surface. The stepped change in wall thickness along the region 13 gives a more gradual change in stiffness and thereby ensures that the catheter curves gradually if it is bent at its patient end. In some catheters, it may be preferable for the inner layer to continue right to the patient tip, but at a reduced thickness. Instead of a stepped change in wall thickness, the inner layer could be gradually reduced in thickness along the region at the patient end.

The construction of the catheter 1 enables it to be made easily by automated processes. The method and machine for making the catheter 1 will now be described with reference to Figure 3.

The extrusion machine 20 has two extrusion heads 21 and 22 for extruding the inner and outer layers 11 and 12 respectively. The rear extrusion head 21 has a conventional hopper 23 and heated extruder screw 24 for supplying the harder plastics material 25 used in the inner layer 11 in molten form to an extrusion die 26. The extrusion die 26 comprises a fixed outer ring 27 having a circular passage 28 defining the outer diameter of the inner layer 11, the passage tapering slightly to a smaller diameter at its forwards, left-hand end. The die 26 also includes a solid inner cone 29 the outside surface of which defines the internal diameter of the inner layer 11. The outer surface of the cone 29 is tapered parallel with the taper on the passage 28 through the outer ring 27 and the cone projects into the ring so that an annular space 30 is defined between the two components, which defines the outlet orifice of the die 26.

The inner cone 29 is connected by a coupling 31 to an actuator control 32 operable to displace the cone axially in either direction. By moving the cone 29 forwards, the internal diameter of the inner layer 11 is increased and its wall thickness reduced: by moving the cone in the opposite direction, the internal diameter of the inner layer is reduced and its wall thickness is increased. This variable internal diameter extruder head could be provided by a Moog valve available from Moog, Inc of East Aurora, NY, USA.

The forward extrusion head 22 has a conventional hopper 33 and heated extruder screw 34 for supplying the softer plastics material 35 used in the outer layer 12 in molten form to an extrusion die 36. The extrusion die 36 comprises a fixed outer ring 37 and a fixed inner ring 38. The inner ring 38 has an aperture of the same diameter as the external diameter of the inner layer 11 so that the ring closely embraces the inner layer as it emerges from the rear extrusion head 21. The outer ring 37 has a circular aperture with a larger diameter equal to the external diameter of the tube 1. A pin 39 may be located between the outer and inner rings 37 and 38 to provide a small diameter lumen within the thickness of the outer layer 12. such as for inflating a cuff. The forward extrusion head 22, therefore, extrudes the softer outer layer 12 directly on top of the harder inner layer 11 so that composite two-layer tubing 40 emerges continuously from the machine 20, as shown in Figure 4, which shows the diameter of the tubing to an exaggerated scale.

The actuator 32 is operated such that the cone 29 is periodically displaced forward to reduce the thickness of the inner layer 11 to about half its usual thickness for a distance of about 13mm and is then displaced fully forwards to reduce the thickness to zero for about 12mm. It is then opened fully to its usual position. In this way, the tubing 40 emerging from the machine 20 will have an inner layer 11 of full thickness along most of its length but interrupted periodically by regions 13 of reduced thickness. The tubing 40 is cut into lengths at the right-hand end of the regions 13 of reduced thickness, which provides the patient end 2 of the finished catheters 1. The cut lengths of tube are then end formed and the side openings are made in the usual way to form the finished catheters 1.

The method of making the catheter 1 enables a soft patient end tip to be provided as a continuous process without the need for subsequent assembly operations.

It will be appreciated that the invention is not confined to epidural catheters but could be used to provide a tip of reduced stiffness to other tubes such as endotracheal tubes. The catheter could be reinforced such as by incorporating a helical reinforcing element, or a braid into the outer layer. A lumen could be formed along the outer layer for various conventional purposes.

## Claims

1. A method of making a medico-surgical tube (1), characterised in that a tubular member is formed by continuously co-extruding an inner layer (11) and an outer layer (12), that the thickness of the extruded inner layer (11) is periodically reduced to form regions (13) along the tubing of reduced thickness, and that the tubing is cut at the regions to form tubes (1) with an end region (13) of reduced stiffness.

2. A method according to Claim 1, characterised in that the thickness of the inner layer (11) is reduced by increasing its internal diameter.

3. A method according to Claim 1 or 2, characterised in that the thickness of the inner layer (11) is reduced along the region (13) such that the stiffness varies along the region.

4. A method according to any one of the preceding claims, characterised in that the thickness of the inner layer (11) is reduced to zero along a part (15) at least of the region (13).

5. A method according to any one of the preceding claims, characterised in that the material (25) of the inner layer (11) is stiffer than the material (35) of the outer layer (12).

6. A method according to any one of the preceding claims, characterised in that the cut end of the tubing including the region (13) of reduced thickness is end formed closed, and that a side opening (3) is formed close to the closed end (2).
